# EUROPEAN PATENT APPLICATION

(11) **EP 1 270 742 A1**
(43) Date of publication of application: **02.01.2003**
(21) Application number: 01915724.7
(22) Date of filing: 23.03.2001
(51) Int. Cl.: C12Q 1/68, C12N 15/22

(54) **METHOD OF DETECTING RISK FACTOR FOR THE ONSET OF ARTERIOSCLEROSIS**

(30) Priority: 24.03.2000 JP 2000084264
(71) Applicant: BML, Inc., Tokyo 151-0051 (JP)
(72) Inventor: NAGANO, Makoto c/o Laboratory, BML, INC., Kawagoe-shi, Saitama 350-1101 (JP); ITO, Mayumi c/o Laboratory, BML, INC., Kawagoe-shi, Saitama 350-1101 (JP); SAGEHASHI, Yukiko c/o Laboratory, BML, INC., Kawagoe-shi, Saitama 350-1101 (JP); HATTORI, Hiroaki c/o Laboratory, BML, INC., Kawagoe-shi, Saitama 350-1101 (JP); EGASHIRA, Toru c/o Laboratory, BML, INC., Kawagoe-shi, Saitama 350-1101 (JP); YAMASHITA, Shizuya, Nishinomiya-shi, Hyogo 663-8174 (JP); MATSUZAWA, Yuji, Takarazuka-shi, Hyogo 665-0804 (JP)
(74) Representative: Bohmann, Armin K., Dr.
(86) International application number: JP0102327
(87) International publication number: WO01071032

(57) **Abstract**

A method of detecting a risk factor for the onset of arteriosclerosis in a subject on the basis of correlation of a gene mutation in the CETP gene (in particular, mutation at (1) guanine at the -69-position of the CETP gene; (2) the site encoding leucine at the 151-position of CETP encoded by the CETP gene; (3) the site encoding arginine at the 201-position and alanine at the 202-position of the same; or (4) the site encoding arginine at the 282-position of the same) with the risk for arteriosclerosis, thereby detecting arteriosclerosis.

SEQUENCE LISTING

<110> BML, INC.

<120> Method of Detecting Risk Factor for Onset of Arteriosclerosis

<130> PBM38/PCT

<140>
<141>

<150> JP P2000-084264
<151> 2000-03-24

<160> 39

<170> PatentIn Ver. 2.1

<210> 1
<211> 2166
<212> DNA
<213> Homo sapiens

<220>
<221> CDS
<222> (28)..(1506)

<400> 1
-360 tgtctttttctcatagtcattgtattttggcctctttctatttatggcaacagagagaga -301

-300 aagcttattcctagatatatgtatttaagtaaaaataaatgaattcatggaaacatatta -241

-240 agcaattatccagataacataagggatggcaaaaatggtgcagatggtggaggggagaca -181

-180 agtagaagttggggtgctcttgttgaatgtctggctctgaactctagaggaggccgcagg -121

-120 ggctgggcaggaaggaggtgaatctctggggccaggaagaccctgctgcccggaagagcc -61

-60 tcatgttccgtgggggctgggcggacatacatatacgggctccaggctgaacggctcggg -1

ccacttacac accactgcct gataacc atg ctg gct gcc aca gtc ctg acc ctg 54


gcc ctg ctg ggc aat gcc cat gcc tgc tcc aaa ggc acc tcg cac gag 102

1 5

gca ggc atc gtg tgc cgc atc acc aag cct gcc ctc ctg gtg ttg aac 150

10 15 20

cac gag act gcc aag gtg atc cag acc gcc ttc cag cga gcc agc tac 198

25 30 35 40

cca gat atc acg ggc gag aag gcc atg atg ctc ctt ggc caa gtc aag 246

45 50 55

tat ggg ttg cac aac atc cag atc agc cac ttg tcc atc gcc agc agc 294

60 65 70

cag gtg gag ctg gtg gaa gcc aag tcc att gat gtc tcc att cag aac 342

75 80 85

gtg tct gtg gtc ttc aag ggg acc ctg aag tat ggc tac acc act gcc 390

90 95 100

tgg tgg ctg ggt att gat cag tcc att gac ttc gag atc gac tct gcc 438

105 110 115 120

att gac ctc cag atc aac aca cag ctg acc tgt gac tct ggt aga gtg 486


125 130 135

cgg acc gat gcc cct gac tgc tac ctg tct ttc cat aag ctg ctc ctg 534

140 145 150

cat ctc caa ggg gag cga gag cct ggg tgg atc aag cag ctg ttc aca 582

155 160 165

aat ttc atc tcc ttc acc ctg aag ctg gtc ctg aag gga cag atc tgc 630

170 175 180

aaa gag atc aac gtc atc tct aac atc atg gcc gat ttt gtc cag aca 678

185 190 195 200

agg gct gcc agc atc ctt tca gat gga gac att ggg gtg gac att tcc 726

205 210 215

ctg aca ggt gat ccc gtc atc aca gcc tcc tac ctg gag tcc cat cac 774

220 225 230

aag ggt cat ttc atc tac aag aat gtc tca gag gac ctc ccc ctc ccc 822

235 240 245

acc ttc tcg ccc aca ctg ctg ggg gac tcc cgc atg ctg tac ttc tgg 870

250 255 260

ttc tct gag cga gtc ttc cac tcg ctg gcc aag gta gct ttc cag gat 918

265 270 275 280

ggc cgc ctc atg ctc agc ctg atg gga gac gag ttc aag gca gtg ctg 966

285 290 300

gag acc tgg ggc ttc aac acc aac cag gaa atc ttc caa gag gtt gtc 1014

300 305 310

ggc ggc ttc ccc agc cag gcc caa gtc acc gtc cac tgc ctc aag atg 1062

315 320 325

ccc aag atc tcc tgc caa aac aag gga gtc gtg gtc aat tct tca gtg 1110

330 335 340

atg gtg aaa ttc ctc ttt cca cgc cca gac cag caa cat tct gta gct 1158

345 350 355 360

tac aca ttt gaa gag gat atc gtg act acc gtc cag gcc tcc tat tct 1206

365 370 375

aag aaa aag ctc ttc tta agc ctc ttg gat ttc cag att aca cca aag 1254

380 385 390

act gtt tcc aac ttg act gag agc agc tcc gag tcc gtc cag agc ttc 1302

395 400 405

ctg cag tca atg atc acc gct gtg ggc atc cct gag gtc atg tct cgg 1350

410 415 420

ctc gag gta gtg ttt aca gcc ctc atg aac agc aaa ggc gtg agc ctc 1398


425                 430                 435                 440

ttc gac atc atc aac cct gag att atc act cga gat ggc ttc ctg ctg     1446

                445                 450                 455

ctg cag atg gac ttt ggc ttc cct gag cac ctg ctg gtg gat ttc ctc     1494

            460                 465                 470

cag agc ttg agc tagaagtctc caaggaggtc gggatggggc ttgtagcaga         1546

        475

aggcaagcac caggctcaca gctggaaccc tggtgtctcc tccagcgtgg tggaagttgg 1606

gttaggagta cggagatgga gattggctcc caactcctcc ctatcctaaa ggcccactgg 1666

cattaaagtg ctgtatccaa gagctgcgga gtccttcttc tgtggctggc gggtagaggg 1726

gggggaaggg gattgtctca ccagtgccgt ccacctcttt tcagcccttc caagcagctg 1786

cccccaaacc ctccaagctt                                               1806

<210> 2
<211> 476
<212> PRT
<213> Homo sapiens

<400> 2
Cys Ser Lys Gly Thr Ser His Glu Ala Gly Ile Val Cys Arg Ile Thr
  1               5                  10                  15

Lys Pro Ala Leu Leu Val Leu Asn His Glu Thr Ala Lys Val Ile Gln
             20                  25                  30

Thr Ala Phe Gln Arg Ala Ser Tyr Pro Asp Ile Thr Gly Glu Lys Ala
        35                  40                  45

Met Met Leu Leu Gly Gln Val Lys Tyr Gly Leu His Asn Ile Gln Ile
    50                  55                  60

Ser His Leu Ser Ile Ala Ser Ser Gln Val Glu Leu Val Glu Ala Lys
65                  70                  75                  80

Ser Ile Asp Val Ser Ile Gln Asn Val Ser Val Val Phe Lys Gly Thr
                85                  90                  95

Leu Lys Tyr Gly Tyr Thr Thr Ala Trp Trp Leu Gly Ile Asp Gln Ser
            100                 105                 110

Ile Asp Phe Glu Ile Asp Ser Ala Ile Asp Leu Gln Ile Asn Thr Gln
        115                 120                 125

Leu Thr Cys Asp Ser Gly Arg Val Arg Thr Asp Ala Pro Asp Cys Tyr
    130                 135                 140

Leu Ser Phe His Lys Leu Leu Leu His Leu Gln Gly Glu Arg Glu Pro
145                 150                 155                 160

Gly Trp Ile Lys Gln Leu Phe Thr Asn Phe Ile Ser Phe Thr Leu Lys
                165                 170                 175

Leu Val Leu Lys Gly Gln Ile Cys Lys Glu Ile Asn Val Ile Ser Asn
            180                 185                 190

Ile Met Ala Asp Phe Val Gln Thr Arg Ala Ala Ser Ile Leu Ser Asp
        195                 200                 205

Gly Asp Ile Gly Val Asp Ile Ser Leu Thr Gly Asp Pro Val Ile Thr
    210                 215                 220

Ala Ser Tyr Leu Glu Ser His His Lys Gly His Phe Ile Tyr Lys Asn
225                 230                 235                 240

Val Ser Glu Asp Leu Pro Leu Pro Thr Phe Ser Pro Thr Leu Leu Gly
                245                 250                 255

Asp Ser Arg Met Leu Tyr Phe Trp Phe Ser Glu Arg Val Phe His Ser
            260                 265                 270

Leu Ala Lys Val Ala Phe Gln Asp Gly Arg Leu Met Leu Ser Leu Met
        275                 280                 285

Gly Asp Glu Phe Lys Ala Val Leu Glu Thr Trp Gly Phe Asn Thr Asn
    290                 295                 300

Gln Glu Ile Phe Gln Glu Val Val Gly Gly Phe Pro Ser Gln Ala Gln
305                 310                 315                 320

Val Thr Val His Cys Leu Lys Met Pro Lys Ile Ser Cys Gln Asn Lys
                325                 330                 335

Gly Val Val Val Asn Ser Ser Val Met Val Lys Phe Leu Phe Pro Arg
            340                 345                 350

Pro Asp Gln Gln His Ser Val Ala Tyr Thr Phe Glu Glu Asp Ile Val
        355                 360                 365

Thr Thr Val Gln Ala Ser Tyr Ser Lys Lys Lys Leu Phe Leu Ser Leu
    370                 375                 380

Leu Asp Phe Gln Ile Thr Pro Lys Thr Val Ser Asn Leu Thr Glu Ser
385                 390                 395                 400

Ser Ser Glu Ser Val Gln Ser Phe Leu Gln Ser Met Ile Thr Ala Val
                405                 410                 415

Gly Ile Pro Glu Val Met Ser Arg Leu Glu Val Val Phe Thr Ala Leu
            420                 425                 430

Met Asn Ser Lys Gly Val Ser Leu Phe Asp Ile Ile Asn Pro Glu Ile

435                 440                 445

Ile Thr Arg Asp Gly Phe Leu Leu Leu Gln Met Asp Phe Gly Phe Pro
    450                 455                 460

Glu His Leu Leu Val Asp Phe Leu Gln Ser Leu Ser
465                 470                 475

<210> 3
<211> 24
<212> DNA
<213> Homo sapiens

<400> 3
cttctgtgct ccagggagga ctca                                              24

<210> 4
<211> 24
<212> DNA
<213> Homo sapiens

<400> 4
cagtttcccc tccagcccac acat                                              24

<210> 5
<211> 22
<212> DNA
<213> Homo sapiens

<400> 5
cagcaaaggc gtgagcctcg tc                                                22

<210> 6
<211> 21
<212> DNA

<213> Homo sapiens

<400> 6
cccaggaatc ctgtctgggc c                                                21

<210> 7
<211> 23
<212> DNA
<213> Homo sapiens

<400> 7
aaaatggtgc agatggtgga ggg                                              23

<210> 8
<211> 24
<212> DNA
<213> Homo sapiens

<400> 8
aatccctata cctggctgtt tgca                                             24

<210> 9
<211> 21
<212> DNA
<213> Homo sapiens

<400> 9
agccctcatc cactgccctc c                                                21

<210> 10
<211> 21
<212> DNA
<213> Homo sapiens

<400> 10
ctgttcctcc cgccacccta c                                                21

<210> 11
<211> 23
<212> DNA
<213> Homo sapiens

<400> 11
cccaacatcc ttcctcactt cca                                              23

<210> 12
<211> 22
<212> DNA
<213> Homo sapiens

<400> 12
agacctcatt cagccctcca cc                                               22

<210> 13
<211> 23
<212> DNA
<213> Homo sapiens

<400> 13
cctggtggta agcattcctg tca                                              23

<210> 14
<211> 23
<212> DNA
<213> Homo sapiens

<400> 14
tggacacact aacaggatgg gca                                              23

<210> 15
<211> 20
<212> DNA
<213> Homo sapiens

<400> 15
caggggctca ttgtggtgct                                                    20

<210> 16
<211> 20
<212> DNA
<213> Homo sapiens

<400> 16
gaccacaggg agtcagccag                                                    20

<210> 17
<211> 22
<212> DNA
<213> Homo sapiens

<400> 17
tgccactccc accttctcca tg                                                 22

<210> 18
<211> 22
<212> DNA
<213> Homo sapiens

<400> 18
gctcccactg ctgtcttctg ag                                                 22

<210> 19
<211> 22
<212> DNA
<213> Homo sapiens

<400> 19
ggttgggtag ctgtgtggat gc                                                22

<210> 20
<211> 23
<212> DNA
<213> Homo sapiens

<400> 20
tgacagaagc cgaataagcc ctc                                               23

<210> 21
<211> 21
<212> DNA
<213> Homo sapiens

<400> 21
acacagggtc cagccagcgt c                                                 21

<210> 22
<211> 21
<212> DNA
<213> Homo sapiens

<400> 22
tccctcctcc ccatgcgtgt g                                                 21

<210> 23
<211> 19

<212> DNA
<213> Homo sapiens

<400> 23
ggactttact ccacccacc                                                    19

<210> 24
<211> 23
<212> DNA
<213> Homo sapiens

<400> 24
ggattggggt acgtgagata aac                                               23

<210> 25
<211> 23
<212> DNA
<213> Homo sapiens

<400> 25
ccgagctact tccttttccc agc                                               23

<210> 26
<211> 22
<212> DNA
<213> Homo sapiens

<400> 26
agtcagttcc cccaccacca cc                                                22

<210> 27
<211> 22
<212> DNA
<213> Homo sapiens

<400> 27
agggttgctc tctgcttcgg ga                                                22

<210> 28
<211> 22
<212> DNA
<213> Homo sapiens

<400> 28
gagttgaccg cccccttctc tt                                                22

<210> 29
<211> 24
<212> DNA
<213> Homo sapiens

<400> 29
ggaagataag accctgccta gata                                              24

<210> 30
<211> 24
<212> DNA
<213> Homo sapiens

<400> 30
cttatttccc ccagtctatc caag                                              24

<210> 31
<211> 22
<212> DNA
<213> Homo sapiens

<400> 31

cttctgtgct ccagggagga ct 22

<210> 32
<211> 19
<212> DNA
<213> Homo sapiens

<400> 32
gggccaggac cgctaagta 19

<210> 33
<211> 22
<212> DNA
<213> Homo sapiens

<400> 33
ccaagaaggg ctgactgggg ct 22

<210> 34
<211> 21
<212> DNA
<213> Homo sapiens

<400> 34
acaccagggt tccagctgtg a 21

<210> 35
<211> 22
<212> DNA
<213> Homo sapiens

<400> 35
cagcacttgg tcatctggtc ac 22

<210> 36
<211> 28
<212> DNA
<213> Homo sapiens

<400> 36
gtaagtggct cgagccgttc agcctgga                                          28


<210> 37
<211> 38
<212> DNA
<213> Homo sapiens

<400> 37
agttggggta cccttgttga atgtctggct ctgaactc                               38


<210> 38
<211> 26
<212> DNA
<213> Homo sapiens

<400> 38
gatgcaactg atacttacac aaccag                                            26


<210> 39
<211> 36
<212> DNA
<213> Homo sapiens

<400> 39
atgcccctga ctgctacctg tctttccata agcagc                                 36

## Description

### Technical Field

The present invention pertains to the technical field relating to methods of detecting disease. More particularly, the present invention relates to a method of detecting arteriosclerosis in a subject.

### Background Art

A great number of epidemiological studies have substantiated that hyperlipidemia is a main risk factor for diseases caused by arteriosclerosis such as cardiac infarction. In terms of cholesterol, a total serum cholesterol level of 240 mg/dL provides a relative risk of coronary heart diseases (CHD) approximately twice that provided by a level of 200 mg/dL. Furthermore, when the total serum cholesterol level reaches 300 mg/dL, the aforementioned risk increases to three fold or greater. Meanwhile, the results of large scale intervention investigations have revealed that the onset of ischemic heart diseases and the number of deaths caused thereby can be reduced by improving hyperlipidemic conditions. On the basis of these facts, in Japan, evaluation of total serum cholesterol level is rendered in three classes: a normal level (less than 200 mg/dL); a boundary level (200-219 mg/dL); and a hypercholesterolemic level (220 mg/dL or more). The Japan Arteriosclerosis Society defines in detail the suggested level of total serum cholesterol at which a drug therapy is employed and the target level to be attained through the therapy, in an addition with the consideration of the presence of other risk factors of CHD.

At present, no one would doubt that hyperlipidemia is a major risk factor for arteriosclerosis, which can lead to lethal cardiac infarction.

The term "hyderlipidemia" encompasses a variety of types of pathological conditions. The most typical is familial hypercholesterolemia (FH), which is caused by poor up-take of serum cholesterol into cells attributable to malfuction of a low-density lipoprotein receptor (LDL-R).

Cholesterol which has been taken into peripheral tissue by the mediation of an LDL-R or a scavenger receptor and excessively accumulated therein is normally trapped by high-density lipoprotein (HDL) and transferred back to the liver, etc. Thus, whether or not the above reverse cholesterol transport pathway functions properly and whether or not cholesterol is properly taken up into cells are important factors to determine the progress of hyperlipidemia or arteriosclerosis.

Cholesteryl ester transfer protein (in the present description, hereinafter may be abbreviated as CETP) and HDL are very closely related to this reverse cholesterol transport pathway.

CETP is a type of protein that exerts an important effect on controlling the quality and quantity of HDL, which prevents arteriosclerosis.

A variety of epidemiological studies have led to wide acceptance of the hypothesis that HDL has an effect of protecting arteriosclerosis. This protective effect relates to the physiological phenomenon that no substantial catabolism of cholesterol occurs in peripheral tissue and that cholesterol must be transferred to the liver by the mediation of plasma lipoproteins in order to be converted to bile acid. Briefly, a possible reason why HDL prevents arteriosclerosis is that HDL takes up excess cholesterol in the peripheral tissue and transfers it to the liver.

Such an effect of HDL has already been confirmed by a variety of animal experiments. For example, experimental animals to which Apo AI―a predominant apolipoprotein of HDL―has been administered and transgenic mice in which human Apo AI has been over-expressed exhibit an increase in serum HDL level, thereby preventing progress of arteriosclerosis caused by a cholesterol-enriched diet.

Furthermore, successive cell-based experiments have revealed anti-arteriosclerosis effect of HDL. For example, preβ-HDL―a sub-fraction of HDL containing almost less lipid components―has been disclosed to "up-take" cholesterol from the peripheral tissue (Fielding, J. *Lipid. Res.* 36: 211-228, 1995).

Thus, at present, there is no doubt that HDL removes cholesterol from peripheral tissue and exerts an anti-arteriosclerotic effect.

In 1965, an experiment in which serum was incubated at 37°C revealed transfer of triglyceride (TG) and cholesteryl ester (CE) between lipoproteins (Nichol *et al., J. Lipid. Res.,* 6: 206-210, 1965). Thereafter, CETP was isolated from human blood plasma as a protein participating in this transfer and was purified to some degree (Chajek *et al., Proc. Natl. Acad. Sci*., U.S.A., 75: 3445-3449, 1978). Currently, perfectly purified CETP has been obtained through subjecting a d>1.21 g/mL fraction isolated from human blood plasma to the following steps: Phenyl-Sepharose column chromatography, CM-Sepharose column chromatography, and DEAE-Sepharose column chromatography, hydroxylapatite column chromatography, etc. Other methods reported for purification of CETP involve steps essentially similar to those outlined above.

CETP is a type of glycoprotein consisting of 476 amino acid residues and has a molecular weight of 64,000-74,000. The variation of the molecular weight reported is attributed to modification of sugar chains corresponding to purification methods. In addition, CETP is known to be a large amount of non-polar amino acids, and highly hydrophobic and to be readily denatured through oxidation.

The human CETP gene is known to be encoded in a length of approximately 25 kb, consisting of 16 exons and 15 introns, and resides in the long arm (16q12-16q21) of the 16th chromosome (Agellon *et al., Biochemistry*, 29: 1372-1376, 1990). It is also known that the cDNA of human CETP has a homology of approximately 80% with that of rabbit CETP.

Excessive cholesterol accumulated in peripheral tissue is trapped by HDL, and esterified on HDL particles through the action of lecithin-cholesterol acyltransferase (LCAT), thereby being transformed to cholesteryl ester (CE). The thus-formed CE is transferred to Apo B-containing lipoproteins, such as very low-density lipoprotein (VLDL), intermediate-density lipoprotein (IDL), and low-density lipoprotein (LDL). Simultaneously, HDL receives TG from these lipoproteins by way of exchange. The resultant CE-rich Apo B-containing lipoproteins are taken into the liver through LDL-R, where these lipoproteins are metabolized. Thus, a so-called reverse cholesterol transport pathway, from the peripheral tissue to the liver, is completed.

In the resultant low-CE, high-TG HDL (HDL₃), TG is decomposed by hepatic triglyceride lipase (HTGL), to thereby form relatively high-apoA-I, low-lipid small HDL particles (preßHDL), conceivably restoring the power to trap cholesterol from the peripheral tissue.

As described above, the reverse cholesterol transport pathway involves a variety of enzymes, such as LCAT, lipoprotein lipase (LPL), and HTGL, and a variety of proteins, such as CETP, phospholipid transfer protein (PLTP), Apo AI, Apo B, and Apo CIII. Among them, CETP plays an important role.

The pathologic and clinical importance of CETP has been elucidated in the cases in which CETP activity is drastically changed, *inter alia,* in "deficient" patients; i.e., patients who are deficient in CETP activity and CETP mass. Yamashita *et al.* have analyzed numbers of patients who are completely CETP-deficient and have obtained the following results (*Atherosclerosis*, 70: 7-12, 1988, *Igaku-no ayumi,* 172: 291-296, 1995).

The serum lipid profile of patients that are completely CETP-deficient exhibits a high total cholesterol level and an HDL cholesterol (HDL-C) level as high as 3-6 times the normal level. In terms of apolipoproteins, Apo AI, Apo CIII, and Apo E levels are elevated considerably, and Apo B level tends to be lowered. An increase in HDL-C is observed exclusively in an HDL₂ fraction. Further detailed analysis of these lipoproteins through electrophoresis has revealed that LDL forms small particles in a polydisperse manner and is poor in CE and that, in contrast, HDL is rich in CE and forms macroparticles thereof. These results indicate that CETP is necessary for the formation of CE-rich, homogeneously matured LDL.

CETP-deficient patients who have a plasma CETP activity and a CETP mass level approximately half those of healthy subjects are heterozygotes and exhibit serum lipid levels, cholesterol levels of a lipoprotein fraction, apolipoprotein levels, and HDL particle sizes which are intermediate values between those of patients who are completely CETP-deficient (homozygotes) and those of healthy subjects. However, some CETP-deficient patients exhibit a normal HDL-C level. In a group including homozygous CETP-deficient patients, heterozygous CETP-deficient patients, and healthy subjects (controls), CETP activity has a negative correlation with the HDL₂ cholesterol level. Thus, the plasma CETP mass level and activity are clearly a major factor for controlling the quantity and quality of HDL.

A study on the interaction between abnormal lipoprotein and cells that is observed in CETP-deficient homozygotes has revealed that CE-depleted Apo B-containing lipoprotein from the CETP-deficient patients has poor affinity to LDL-Rs existing in fibroblasts as compared with normal Apo B-containing lipoprotein (Sakai N., *Eur. J. Clin. Invest.,* 25: 332-339, 1995). Regarding HDL, CE-rich large HDL particles observed in CETP-deficient patients have a considerably reduced effect on macrophages for preventing formation of foam cells and for defoaming the foam cells (Ishigami M., *J. Biochem.,* 116: 257-262, 1994). In addition, large HDL particles which are observed in CETP-deficient patients are caused to disappear by addition of purified CETP, thereby transforming into VHDL-like small particles smaller than HDL₃ particles (Yamashita S., *Ann. NY Acad. Sci*. U.S.A., 748: 606-608, 1995). The analytical results of the above VHDL-like particles purified by gel filtration reveal that the particles contain phospholipid and Apo AI as a predominant apolipoprotein; strongly prevent the foam cell formation in macrophages induced by acetylated LDL; and strongly promote defoamation of foam cells in macrophages in which cholesterol has already been accumulated. Briefly, CETP plays a role in restoring functions of HDL through its CE transfer among lipoproteins, in which has lost the ability to prevent formation of foam cells, and also the ability to defoam the cells.

In all cases, these results indicate that CETP deficiency is disadvantageous in the reverse cholesterol transport pathway.

A variety of transgenic mice to which the human CETP gene has been transferred have been created, and the lipid metabolism in such transgenic mice has been investigated.

CETP in mouse is essentially lacking, and mouse lipoprotein predominantly contains HDL, which is considerably different from that of humans. Thus, the investigation in lipid metabolism in mice must be very carefully adapted to the human case; nonetheless, studies involving mice have yielded a variety of useful findings for elucidating CETP function.

Agellon *et al*. transferred the human CETP gene into mice (CETP-Tg), induced expression of the gene, and observed changes in HDL-C level (*J. Biol*. *Chem.,* 266: 10796-10801, 1991). The CETP-Tg group exhibits reduction in HDL-C level by 15-27% under administration of a normal diet. This tendency is more marked under administration of a high-fat diet.

Hayek *et al.* have crossbred a transgenic mouse for expressing human Apo AI (A-I-Tg) and a transgenic mouse for expressing human CETP (CETP-Tg), to thereby produce A-I/CETP-Tg mouse. The effect of CETP on HDL-C level and HDL particle size has been investigated in this A-I/CETP-Tg mouse (*J. Clin*. *Invest.,* 90: 505-510, 1992). The CETP-Tg group exhibits a remarkable reduction in HDL-C level as compared with non-Tg mice, and also a reduction in HDL particle size. The A-I/CETP-Tg group also exhibits this tendency, but to a more remarkable extent. Moreover, transfer of CE to Apo B-containing lipoprotein is promoted in all mice in the CETP-Tg group, thereby indicating enhancement in turnover of HDL.

Jiang *et al*. have studied the relationship between CETP and LDL-R by regulating the expression level of CETP-Tg (*J. Biol. Chem.,* 268: 27406-27412, 1993). The results indicate that concentrations of serum VLDL-C, LDL-C, and Apo B increase corresponding to the increased serum CETP concentration. In the case of the CETP-Tg, the amount of LDL-R mRNA in the liver decreases to a maximum of approximately 1/2 that found in non-Tg mice, and a negative correlation has been identified between the serum CETP level and the amount of LDLR mRNA. Also in the case of the CETP-Tg, the cholesterol and CE content increase in the liver, whereas in contrast, the mRNA expression of HMG-CoA reductase and cholesterol 7α-hydroxylase decreases in the liver. These results are conceivably caused by the mechanism that CE transfer to VLDL and LDL is promoted by over-expression of CETP, leading to an increase in amount of cholesterol taken up to the liver, thereby finally leading to down-regulation of hepatic LDL-R.

As described above, a variety of experiments have revealed that, in the living body, CETP functions to accelerate the reverse cholesterol transport pathway.

Yamashita *et. al* have conducted an epidemiological study in Omagari-city, Akita, Japan, where incidence of high-HDL cholesterolemia (hyperalphacholesterolemia) with low-CETP activity are quite high (Hirano K. *et al., Arterioscler. Thromb. Vasc. Biol*., 17(6): 1053-1059, 1997).

In that region, the incidence of hyperalphacholesterolemia is considerably higher than in other regions. This clearly coincides with the finding that the incidence of CETP deficiency caused by abnormality in splicing at intron 14 of the CETP gene is found to be 5-8 times that of other regions. The average life expectancy of the people living in the region is slightly shorter than that of all Japanese, and the incidence of hyperalphacholesterolemia with CETP deficiency in a group of people aged 80 or more is rather low as compared with a group of people under age 80. Furthermore, investigation on the relationship in this region between the HDL-C level and the incidence of subjects with ischemic changes in electrocardiograms at rest has revealed that the incidence occurs the lowest at an HDL-C level of about 60 mg/dL and increases as the HDL-C level increases from this level. Therefore, from the cases of hyperalphacholesterolemia caused by CETP deficiency in this region, it has been considered that hyperalphacholesterolemia does not mean resistance to arteriosclerosis, but rather indicates a converse effect to arteriosclerosis.

A. R. Tall (Columbia University, U.S.A.) *et al*. have carried out epidemiological studies on CETP-deficient Americans of Japanese parentage and mentioned that the cases of coronary heart diseases are recognized more significantly in CETP-deficient subjects than in non-CETP-deficient subjects (Zhong S. *et al., J. Clin, Invest.* 15: 97(12): 2917-2923, 1996).

Hitherto, mutations in the CETP gene to cause CETP deficiency and the polymorphism of the CETP gene that manifests functional changes have been reported mainly by Japanese researchers. Specific examples of nonsense mutation include G181X (glycine at amino acid residue 181 (Gly (GGA)) encoded by exon 6 serves as a stop codon (TGA)) (Arai T., *J. Lipid Res.,* 37: 2145-2154, 1996) and Q309X (glutamine at amino acid residue 309 (Gln (CAA)) encoded by exon 10 serves as a stop codon (TAA)) (Gotoda T., *Biochem. Blophys. Res. Commun*., 194: 519-524, 1993). Examples of nonsense mutation involving splicing abnormality include substitution of glycine (G) at the 1st base of the intron 14 splice donor site by adenine (A) (Int14 + 1G → A) (Brown M., *Nature*, 342: 448-451, 1989; Yamashita S., *Biochem. Biophys. Res. Commun.,* 170: 1346-1351, 1991); insertion of T after thymine (T) at the 2nd base of the intron 14 splice donor site (Int14 + 3Tins) (Inazu A., *J. Clin. Invest.,* 94(5): 1872-1882, 1994); and substitution of T at the 2nd base of the intron 10 splice donor site by G (Int10 + 2T → G) (Sakai N., *J. Lipid Res.,* 37: 2065-2073, 1996).

Examples of polymorphism in terms of function conceivably resulting in reduction in CETP activity include D442G (substitution of asparagine at amino acid residue 442 (Asp (GAC)) encoded by exon 15 by glycine (Gly (GGC))) (Taskahashi K., *J. Clin. Invest.,* 92: 2060-2064, 1993); I405V; and R451Q. However, physiological and functional changes have not yet been clearly elucidated. In addition, polymorphism cases such as intron 1 TaqlB polymorphism (Freeman D. J., *Arterioscler. Thromb.,* 14: 336-344, 1994), which is conceivably concerned with variation in serum CETP mass level, have been reported.

Among the cases of the CETP mutation causing CETP deficiency, a mutation of Int14 + 1G → A occurs at the highest incidence. This mutation has been investigated among Japanese citizens (Hirano K., *Arteriosclerosis,* 100: 85-90, 1993), revealing that in a group in which HDL-C level was 100 mg/dL or higher, 6 out of 171 cases (3.5%) were homozygotes in which both alleles were A-mutated, and 48 cases (28.1%) were heterozygotes in which one allele was A-mutated, with gene frequency of A being 0.1754. Also, in a group of 89 cases in which HDL-C level was 60 mg/dL or higher and lower than 100 mg/dL, no homozygote was identified and only 11 cases (12.4 %) were heterozygotes, with gene frequency of A being as low as 0.0618. In a general group of 512 cases, no homozygote was identified, and only 5 cases (0.98 %) were heterozygotes, this percentage being apparently small, and gene frequency of A was considerably low; i.e., as low as 0.0049. These results indicate that mutation in the CETP gene is closely related to expression of CETP and serum HDL-C level.

However, manifestation of other mutations, such as G181X, Q309X, Int10 + 2T → G, and Int14 + 3Tins, are quite rare.

Although about half the patients who are completely CETP deficient where no serum CETP is detected can be identified as homozygotes of Int14 + 1G → A mutation, only other known mutations cannot account for the remaining patients. In addition, other types of introngenic mutation and mutation in a transcription regulatory region have rarely been reported.

A variety of investigations of clinical profiles of D442G polymorphism have been performed. The frequency of emergence thereof is approximately 7% in a general group and is conceived to be elevated to approximately 30% in a group in which HDL-C level is 80 mg/dL or higher. In addition, clinical profiles of I405V polymorphism and R451Q polymorphism and the emergence frequency thereof have also been investigated. However, the relation on these types of polymorphism to diseases has not been clearly elucidated.

As described above, the relationship between a gene mutation and abnormality in a specific function of CETP or in the CETP mass level have been investigated extensively, and elucidated to a certain extent. However, the relationship cannot be completely described on the basis of currently reported Int14 + 1G → A type mutation and other types of mutation. In addition, at present, the mechanism causing a low serum CETP level for causing hyperalphacholesterolemia cannot be completely described on the basis of mutations in the CETP gene and other factors such as enzymes or proteins in the body other than controlling of the CETP gene.

Thus, an object of the present invention is to identify a novel, high-frequent gene mutation which causes CETP deficiency. Another object is to identify means for preventing and retarding the development of arteriosclerosis on the basis of the finding on the mutation.

### Disclosure of the Invention

In order to attain the aforementioned objects, the present inventors have conducted earnest studies on the identification of a novel, high-frequent gene mutation causing CETP deficiency.

Specifically, CETP gene analysis was performed for 13 hyperalphacholesterolemic cases which exhibited a clear reduction in serum CETP activity to 40% or less as compared with subjects having a normal serum HDL-C level.

As a result, the present inventors have found that the gene mutation of interest is identified at (1) guanine at the -69-position of the CETP gene; (2) the site encoding leucine at the 151-position of CETP encoded by the CETP gene; (3) the site encoding arginine at the 201-position and alanine at the 202-position of the same; or (4) the site encoding arginine at the 282-position of the same. The inventors have also found that a risk factor for the onset of arteriosclerosis in a subject can be detected on the basis of the relevance of the novel types of mutation and/or conventionally identified mutation to the risk of arteriosclerosis.

Accordingly, in the present invention, the inventors provide a method of detecting arteriosclerosis; i.e., a risk factor for the onset of arteriosclerosis in a subject on the basis of the relevance of mutation in the CETP gene to the risk of arteriosclerosis (hereinafter referred to as a detection method of the present invention). Particularly, employment of one or more types of mutation identified at sites selected from among (1) guanine at the -69-position of the CETP gene; (2) the site encoding leucine at the 151-position of CETP encoded by the CETP gene; (3) the site encoding arginine at the 201-position and alanine at the 202-position of the same; and (4) the site encoding arginine at the 282-position of the same is important for the detection method of the present invention.

In the present specification, amino acids are represented by three-letter codes and one-letter codes. For the sake of assuredness, the following illustrate specific representations.

"Alanine [Ala (three-letter code, the same applies to the following), A (one-letter code, the same applies to the following)]; valine [Val, V]; leucine [Leu, L]; isoleucine [Ile, I]; proline [Pro, P]; phenylalanine [Phe, F]; tryptophan [Trp, W]; methionine [Met, M]; glycine [Gly, G]; serine [Ser, S]; threonine [Thr, T]; cysteine [Cys, C]; glutamine [Gln, Q]; asparagine [Asn, N]; tyrosine [Tyr, Y]; lysine [Lys, K]; arginine [Arg, R]; hystidine [His, H]; aspartic acid [Asp, D]; and glutamic acid [Glu, E]."

In the present specification, for example, "A100V" refers to a mutation in which alanine at the 100-position of the authentic amino acid sequence is substituted by valine.

### Brief Description of the Drawings

Fig. 1 shows the results of identification of a mutation Int14 + 1G → A in the CETP gene by PCR-RFLP.
Fig. 2 shows the results of identification of a mutation D442G in the CETP gene by PCR-RFLP.
Fig. 3 shows the partial nucleotide sequence of transcription regulatory region in the CETP gene of case 11 obtained by direct sequencing.
Fig. 4 shows the partial nucleotide sequences of exons 5 in the CETP gene of case 12 obtained by direct sequencing.
Fig. 5 shows the partial nucleotide sequence of sub-cloned exon 7 in the CETP gene of case 9 obtained by sequencing.
Fig. 6 shows the partial nucleotide sequence of exon 9 in the CETP gene of case 13 obtained by direct sequencing.
Fig. 7 shows the results of analysis concerning the effect of a mutation -69G → A in a CETP gene on transcription activity.
Fig. 8 shows the results of identification of a mutation -69G → A in the CETP gene by PCR-RFLP.
Fig. 9 shows the results of identification of a mutation L151P in the CETP gene by PCR-RFLP.
Fig. 10 shows the results of identification of a mutation 680 del GGG/ins AAACG in the CETP gene by PCR-RFLP.
Fig. 11 shows the results of identification of a mutation R282C in the CETP gene by PCR-RFLP.

### Best Modes for Carrying Out the Invention

Modes for carrying out the present invention will next be described.

As mentioned above, the present invention is directed to a method of detecting arteriosclerosis by detecting a risk factor for the onset of arteriosclerosis on the basis of the detection of gene abnormalities in the CETP gene, which is a gene relating to arteriosclerosis.

A CETP gene and the CETP encoded by the CETP gene have already been analyzed (Agellon *et al., Biochemistry,* 29: 1372-1376, 1990, DDBJ/EMBL/GenBank database ACCESSION M32992 J02898 - M32998 J02898). The CETP gene sequence and the amino acid sequence of CETP are shown in SEQ ID Nos. 1 and 2, respectively.

As is also described above, cDNA of the human CETP is known to have a homology of approximately 80% with that of rabbit CETP.

Through detailed analysis of correlation between mutations in the CETP gene and phenomena induced by abnormalities of CETP, such as high-HDL cholesterolemia (hyperalphacholesterolemia), the mutations in the CETP gene that are employable in the detection method of the present invention can be identified. In other words, desired mutations can be identified through analysis of, for example, "patients exhibiting high-HDL cholesterolemia (hyperalphacholesterolemia) and healthy subjects" in combination, in terms of the mutation sites and frequency in the CETP gene and a function of protein provided by the mutation. The actual operations of the analysis will be described specifically in the below-mentioned "Examples."

In the present invention, the term "gene mutation" (or simply "mutation") refers to a genetic mutation occurring in human chromosomes; i.e., the case in which the nucleotide sequence of the gene is different from that of a wild type (the nucleotide sequence of a normal gene). Cases where a gene has a particular site in the nucleotide sequence thereof and variation from individual to individual is observed at that particular site are generally called "polymorphism." In the present invention, "gene mutation" also encompasses "polymorphism." The "gene mutation" is characterized through a wide range of analyses in terms of mutation frequency, amount of expressed mRNA, amount of expressed protein, function of the protein, etc. Such a "gene mutation" is conceived to be present at a rate of approximately one per some hundreds nucleotides on average, and the gene mutation can be identified through direct or indirect analysis thereof. In addition, through analysis of the genealogy of the identified mutation, a determination can be made as to whether the mutation is derived from a paternal allele or a maternal allele.

Variations occurring in a mutation site are provided by either a paternal gene or a maternal gene having a mutation site where a base of the nucleotide sequence is substituted. When the genes of both alleles have undergone substitution of a base with another base different from a relevant base of a wild type nucleotide sequence of the genes, the individual is identified as a "homozygote", whereas when the gene of one allele has undergone substitution of a base with another base different from a relevant base of a wild type nucleotide sequence of the gene, the individual is identified as a "heterozygote".

As described below, the present inventors have identified the following gene abnormalities which occur in the CETP gene and are considered to have correlation to a risk factor for the onset of arteriosclerosis: (1) a mutation at guanine at the -69-position of the CETP gene (e.g., a mutation specified by the presence or absence of sensitivity to the restriction enzyme *Hap*II in a transcription regulatory region); (2) a mutation at the site encoding leucine at the 151-position of CETP encoded by the CETP gene (e.g., a mutation specified by the presence or absence of sensitivity to restriction enzyme *Alu*I in the exon 5 region when a mismatch primer represented by SEQ ID NO: 39 is used); (3) a mutation at the site encoding arginine at the 201-position and alanine at the 202-position of the same (e.g., a mutation specified by sensitivity to restriction enzyme *Xcm*I in the exon 7 region); and (4) a mutation at the site encoding arginine at the 282-position of the same (e.g., a mutation specified by the presence or absence of sensitivity to restriction enzyme *Hae*III in the exon 9 region).

Variations in genetically abnormal site are detected through any known method, and examples of methods that can be employed include Southern blotting-RFLP, PCR-RFLP, the HET (heteroduplex analysis) method, the DGGE (denaturing gradient gel electrophoresis) method, the DS (direct sequence) method, the CCM (chemical cleavage mismatch) method, the CDI (carbodiimide modification) method, the PCR-SSCP (single-stranded conformation polymorphism) method (in the present specification, hereinafter referred to as the SSCP method), and the PCR/GC-clamp method (see, e.g., Biomanual Series 1, Basic technique of Genetic Engineering, edited by Tadashi Yamamoto, Yodo-sha (1993), and particularly for the PCR/GC-clamp method, see Myers, R. M., Shefield, V., and Cox, D. R. (1988) in Genomic Analysis: A Practical Approach. K. Davies, ed. IRL Press Limited, Oxford, pp. 95-139, etc.). Of these, the PCR/GC-clamp method is preferably employed, in that abnormalities of a gene can be determined correctly and conveniently.

The PCR/GC-clamp method is a variation of the DGGE method―a method for detecting base substitution in DNA employing the difference in mobility between a double-stranded DNA fragment including base substitution and a double-stranded DNA fragment free of base substitution which are placed in polyacrylamide gel having a proportional gradation in concentration of a DNA modifier, the difference being induced by difference in concentration of the DNA modifier for modifying two types of DNA fragments. One drawback of the DGGE method is that base substitution in the domain finally melted in polyacrylamide gel cannot be detected when a plurality of base substituted portions are generated. The PCR/GC-clamp method overcomes this drawback by linking a domain of high GC content (GC-clamp) to a DNA fragment for which base substitution is detected [see Shefield, V. C. *et al.* (1989) *Proc. Natl. Acad. Sci.* USA 86: 232-236, etc.].

Thus, the PCR/GC-clamp method is similar to the DGGE method in terms of operation, etc., but requires a step for linking a GC-clamp to a DNA fragment for which base substitution is detected.

In the detection method of the present invention, no particular limitation is imposed on the origin of DNA for detecting a variation in an abnormal site in the CETP gene, and any cells can be used as a DNA source so long as the cells have been sampled from the subject. For example, blood samples such as peripheral blood and leucocytes are preferably selected in the present invention.

Genomic DNA is extracted from a cell specimen sampled from the subject through a known method. By use of the genomic DNA, variation in a specific site in the gene (specifically, base substitution at a specific abnormal site in the gene) is detected.

If the above detection procedure detects any variation at the aforementioned specific site in the gene, a risk factor for the onset of arteriosclerosis can be detected on the basis of correlation between the variation at the site of the gene and the risk of arteriosclerosis. The expression "detection of a risk factor for the onset of arteriosclerosis" refers to a concept encompassing detection of a high probability of developing arteriosclerosis as well as detection of currently occurring arteriosclerosis. In other words, if any variation is identified at a specific site in a CETP gene, the intrinsic role of CETP cannot fully be attained during serum lipid metabolism or other processes, to thereby elevate the probability of developing arteriosclerosis, even when no arteriosclerosis is currently identified. According to the detection method of the present invention, such high probability of developing arteriosclerosis can be detected as a risk factor for the onset of arteriosclerosis.

When CETP does not play its intrinsic role, a number of cases in which HDL-C level is high among other serum lipids are found. Generally, HDL-C is recognized as "good" cholesterol, and the probability of the onset of arteriosclerosis is not usually considered to be high even though the HDL-C level among other serum lipids is high. By performing the detection method of the present invention, a risk factor for the onset of arteriosclerosis hiding behind high HDL-C level can be detected for a subject who exhibits a high HDL-C level among other serum lipid levels while no apparent risk factor for the onset of arteriosclerosis is identified.

When the detection method of the present invention identifies a variation at the aforementioned specific site in the CETP gene of a subject and diseases closely related to arteriosclerosis such as diabetes, coronary heart diseases, and hypertension are identified in the subject, the risk of arteriosclerosis in the subject can be reduced by taking a medication of treatment for ameliorating arteriosclerosis as well as general treatment for the above diseases. Even when a variation is identified at the aforementioned specific site in the CETP gene of a subject through the detection method in the present invention, if the subject appears to be healthy, the risk factor for the onset of arteriosclerosis of the subject can be made less serious by taking measures for preventing the onset of arteriosclerosis in advance; e.g., guidance and control of diet and physical exercise.

In the detection method of the present invention, accuracy of the detection of a risk factor for the onset of arteriosclerosis can be enhanced by detecting, in combination, an abnormality at a specific site in the CETP gene identified according to the present invention and conventionally identified abnormalities at specific sites in the CETP gene such as the aforementioned G181X, Q309X, Int10 + 2T → G, Int14 + 1G → A, Int14 + 3Tins, and D442G.

### Examples

The present invention will next be described in more detail by way of examples, which should not be construed as limiting the technical scope of the invention thereto.

### 1. CETP gene analysis

### Subjects

CETP gene analysis was performed for 13 hyperalphalipoproteinemic patients who have proven to exhibit a serum CETP activity reduced to 40% or less that found in subjects having a normal serum HDL-C level (i.e., subjects having serum lipid levels falling within a normal range, the same applies to the following) and who had agreed to undergo the gene analysis according to the present invention.

### Measurement of serum CETP activity

The CETP activity was measured in accordance with a method described by Kato, H. *et al*. (*J. Biol. Chem.,* 264: 4082-4087, 1989) employing re-constituted HDL. Specifically, donor lipoprotein (re-constituted HDL) having an RI-labeled cholesteryl ester (CE) moiety and acceptor lipoprotein (LDL) were mixed at predetermined proportions, and serum was added to the mixture. The resultant mixture was allowed to react for a predetermined period of time. Subsequently, the donor lipoprotein and the acceptor lipoprotein were separated from each other by a dextran sulfate precipitation method. Radioactivity transferred to the acceptor lipoprotein was measured. Detailed procedures of the measurement will be described below.

### Preparation of LDL

LDL (density: 1.019 < d < 1.063) was prepared from pooled human plasma (400 mL) through ultracentrifugation. Specifically, KBr (0.01851 g per mL of EDTA-added plasma) was added to EDTA-added plasma, to thereby adjust the specific gravity of the mixture at d=1.019, and the mixture was centrifuged at 45,000 rpm and 12°C for 16 hours (rotor: 50.2 Ti, centrifuge: model L-80, product of BECKMAN). After completion of centrifugation, a fraction provided in the upper layer of the tube and having a specific gravity lower than that of IDL was removed. The lower layer (approximately 245 mL) was collected, and KBr (0.0644 g per mL of the lower layer) was added to the lower layer, to thereby adjust the specific gravity of the mixture at d=1.063. The mixture was subjected to ultracentrifugation at 45,000 rpm and 12°C for 20 hours. After completion of centrifugation, the uppermost layer in the tube, assuming an intense orange color and containing LDL, was collected. The collected LDL-containing layer was dialyzed against PBS (pH 7.4) containing 0.01% NaN₃ (2 L) at 4°C for 12 hours, and this procedure was repeated three times. LDL fractions were separated from the thus-dialyzed product through a filter (0.45 µm) and sterilized. Protein in the separated LDL fractions was quantitated through the Lowry's method (Lowry, O. H. *et al., J. Biol. Chem.,* 193: 265-275, 1951), and the protein content was adjusted to 3.25 mg/mL. The thus-prepared LDL was stored at 4°C under sterilized conditions.

### Purification of Apolipoprotein A-I

Apo A-I was obtained through purification of pooled human plasma (500 mL) by use of CIBACRON BLUE 3GA Type 3000-CL (product of SIGMA) and DEAE Sepharose CL-6B (product of PHARMACIA). The thus-purified Apo A-I was defatted, followed by lyophilization. Subsequently, the lyophilized Apo A-I was dissolved in PBS (pH 7.4) containing 6M urea and the solution was dialyzed against PBS (pH 7.4) containing 0.1% sodium cholate (2 L) at 4°C for 12 hours (dialysis was performed a total of three times). After completion of dialysis, protein was quantitated through the Lowry's method.

### Production of re-constituted HDL

Cholesterol (5.625 µmol, product of SIGMA), cholesteryl oleate (0.47 µmol, product of SIGMA), phosphatidylcholine (22.5 µmol, product of NAKALAI TESQUE), and cholesteryl 1-¹⁴C-labeled-oleate (product of AMERSHAM) were placed in a glass tube, and the mixture was dried under a stream of nitrogen. The dried mixture was dissolved in ethanol (1 mL). In another glass tube, PBS (20 mL) was placed and vigorously stirred by means of a stirrer (product of CORNING) under a strong stream of nitrogen. Into this glass tube, the above-prepared ethanol solution (1 mL) was injected by use of a 26G syringe (product of TERUMO). The stirring speed was reduced to half, and stirring was continued for five minutes, following by addition of 200 mM sodium cholate (1.9 mL) and additional stirring for two minutes. At this stage, the apolipoprotein A-I purified in the aforementioned manner (18 mg) was added to the mixture, and stirring was continued for two minutes. PBS was added to the resultant mixture, to thereby adjust the total volume to 25 mL. The mixture was dialyzed against PBS (pH 7.4) containing 0.01% NaN₃ (2 L) at 4°C for 12 hours, and dialysis was performed a total of three times, to thereby produce re-constituted HDL.

### Measurement of CETP activity

The re-constituted HDL prepared in the aforementioned manner (50 µL), 3.25 mg/mL LDL (20 µL), and a serum sample (80 µL) that had been diluted 40-fold by use of PBS were mixed, and the mixture was incubated at 37°C for 30 minutes for allowing the mixture to react. Subsequently, ice-cooled 0.1% dextran sulfate (15 µL) and 60 mM magnesium chloride (15 µL) were added to the reaction mixture, followed by vigorous voltexing. The resultant mixture was allowed to stand on ice for 20 minutes, and centrifuged at 12,000 rpm at 4°C for 10 minutes by a centrifuge (model MRX-151, product of TOMY). The resultant supernatant was separated out, and the precipitated LDL was dissolved in 0.1N NaOH (180 µL). From the solution, an aliquot of 150 µL was sampled, and radioactivity of the samples was measured by means of a liquid scintillation counter (model LS5000TD, product of BECKMAN). CETP activity of each subject was obtained as a relative percent of CE transfer obtained on the concurrently measured average percent of CE transfer in control serum of healthy subjects (subjects exhibiting normal serum HDL-C level) as 100%. Upon this CETP measurement, CETP activity of 49 subjects (26 male, 23 female)with normal serum HDL-C level was found to be 100 ± 19% (average ± standard deviation).

The following Table shows serum CETP activity and HDL-C level of 13 hyperalphalipoproteinemic subjects.

**Table 1**

| Case No. | Sex | CETP activity (%) | HDL-C (mg/dL) |
|---|---|---|---|
| 1 | F (female) | 0 | 147 |
| 2 | M (male) | 0 | 117 |
| 3 | M | 0 | 152 |
| 4 | M | 0 | 208 |
| 5 | F | 1 | 171 |
| 6 | F | 1 | 124 |
| 7 | M | 1 | 152 |
| 8 | M | 2 | 210 |
| 9 | M | 3 | 164 |
| 10 | M | 25 | 122 |
| 11 | M | 30 | 110 |
| 12 | M | 31 | 106 |
| 13 | M | 40 | 136 |

### Extraction of genomic DNA

Through use of a QIAamp Blood Kit (product of QIAGEN), genomic DNA of hyperalphalipoproteinemia was extracted from peripheral blood of each subject collected with a vacuum blood collection tube containing an anticoagulant EDTA-3K.

### Arrangement of primers

A gene encoding human CETP (SEQ ID NO: 1) and the amino acid sequence of the protein (SEQ ID NO: 2) were procured from DDBJ/EMBL/GenBank Data Base ACCESSION M32992 J02898 - M32998 J02898 (Agellon, L. B., *et al.*).

The nucleotide sequence of respective exons of the CETP gene are represented as follows; exon 1: from the 388 position to the 505 position of SEQ ID NO: 1; exon 2: from the 506 position to the 620 position of SEQ ID NO: 1; exon 3: from the 621 position to the 755 position of SEQ ID NO: 1; exon 4: from the 756 position to the 826 position of SEQ ID NO: 1; exon 5: from the 827 position to the 914 position of SEQ ID NO: 1; exon 6: from the 915 position to the 984 position of SEQ ID NO: 1; exon 7: from the 985 position to the 1045 position of SEQ ID NO: 1; exon 8: from the 1046 position to the 1137 position of SEQ ID NO: 1; exon 9: from the 1138 position to the 1317 position of SEQ ID NO: 1; exon 10: from the 1318 position to the 1368 position of SEQ ID NO: 1; exon 11: from the 1369 position to the 1533 position of SEQ ID NO: 1; exon 12: from the 1534 position to the 1601 position of SEQ ID NO: 1; exon 13: from the 1602 position to the 1635 position of SEQ ID NO: 1; exon 14: from the 1636 position to the 1708 position of SEQ ID NO: 1; exon 15: from the 1709 position to the 1794 position of SEQ ID NO: 1; and exon 16: from the 1795 position to the 1869 position of SEQ ID NO: 1.

Arrangement of primers for PCR amplification of each exon was performed by use of a computer software (GENETYX-MAC, product of Software Kaihatsu).

The nucleotide sequences of the PCR primers are described as below.

### For analysis of mutation in a CETP gene

### Int14 + 1G → A mutation

5'-CTT CTG TGC TCC AGG GAG GAC TCA-3' (Forward: SEQ ID NO: 3)

5′-CAG TTT CCC CTC CAG CCC ACA CAT-3′ (Reverse: SEQ ID NO: 4)

*Underlined: mismatch base

### D442G mutation

5'-CAG CAA AGG CGT GAG CCT CGT C-3' (Forward: SEQ ID NO: 5)

5′-CCC AGG AAT CCT GTC TGG GCC-3′ (Reverse: SEQ ID NO: 6)

*Underlined: mismatch base

### For sequence analysis

### Exon 1

5'-AAA ATG GTG CAG ATG GTG GAG GG-3' (Forward: SEQ ID NO: 7)

5′-AAT CCC TAT ACC TGG CTG TTT GCA-3′ (Reverse: SEQ ID NO: 8)

### Exon 2

5′-AGC CCT CAT CCA CTG CCC TCC-3′ (Forward: SEQ ID NO: 9)

5'-CTG TTC CTC CCG CCA CCC TAC-3'   (Reverse: SEQ ID NO: 10)

### Exon 3

5′-CCC AAC ATC CTT CCT CAC TTC CA-3′ (Forward: SEQ ID NO: 11)

5′-AGA CCT CAT TCA GCC CTC CAC C-3′ (Reverse: SEQ ID NO: 12)

### Exons 4 and 5

5'-CCT GGT GGT AAG CAT TCC TGT CA-3' (Forward: SEQ ID NO: 13)

5′-TGG ACA CAC TAA CAG GAT GGG CA-3′ (Reverse: SEQ ID NO: 14)

### Exon 6

5′-CAG GGG CTC ATT GTG GTG CT-3′ (Forward: SEQ ID NO: 15)

5'-GAC CAC AGG GAG TCA GCC AG-3' (Reverse: SEQ ID NO: 16)

### Exon 7

5'-TGC CAC TCC CAC CTT CTC CAT G-3' (Forward: SEQ ID NO: 17)

5′-GCT CCC ACT GCT GTC TTC TGA G-3′ (Reverse: SEQ ID NO: 18)

### Exon 8

5'-GGT TGG GTA GCT GTG TGG ATG C-3' (Forward: SEQ ID NO: 19)

5′-TGA CAG AAG CCG AAT AAG CCC TC-3′ (Reverse: SEQ ID NO: 20)

### Exon 9

5′-ACA CAG GGT CCA GCC AGC GTC-3′ (Forward: SEQ ID NO: 21)

5′-TCC CTC CTC CCC ATG CGT GTG-3′ (Reverse: SEQ ID NO: 22)

### Exon 10

5′-GGA CTT TAC TCC ACC CAC C-3′ (Forward: SEQ ID NO: 23)

5′-GGA TTG GGG TAC GTG AGA TAA AC-3′ (Reverse: SEQ ID NO: 24)

### Exon 11

5′-CCG AGC TAC TTC CTT TTC CCA GC-3′ (Forward: SEQ ID NO: 25)

5′-AGT CAG TTC CCC CAC CAC CAC C-3′ (Reverse: SEQ ID NO: 26)

### Exon 12

5′-AGG GTT GCT CTC TGC TTC GGG A-3′ (Forward: SEQ ID NO: 27)

5′-GAG TTG ACC GCC CCC TTC TCT T-3′ (Reverse: SEQ ID NO: 28)

### Exon 13

5′-GGA AGA TAA GAC CCT GCC TAG ATA-3′ (Forward: SEQ ID NO:
29)

5′-CTT ATT TCC CCC AGT CTA TCC AAG-3′ (Reverse: SEQ ID NO:
30)

### Exon 14

5′-CTT CTG TGC TCC AGG GAG GAC T-3′ (Forward: SEQ ID NO: 31)

5′-GGG CCA GGA CCG CTA AGT A-3′ (Reverse: SEQ ID NO: 32)

### Exons 15 and 16

5′-CCA AGA AGG GCT GAC TGG GGC T-3′ (Forward: SEQ ID NO: 33)

5′-ACA CCA GGG TTC CAG CTG TGA-3′ (Reverse: SEQ ID NO: 34)

### For measurement of CETP transcription activity

### pCETP-570 vector

5'-CAG CAC TTG GTC ATC TGG TCA C-3' (Forward: SEQ ID NO: 35)

5′-GTA AGT GGC TCG AGC CGT TCA GCC TGG A-3′ (Reverse: SEQ ID
NO: 36)

*Underlined: site recognized by *Xho*I

### pCETP-164 vector

5′-AGT TGG GGT ACC CTT GTT GAA TGT CTG GCT CTG AAC TC-3′
(Forward: SEQ ID NO: 37)

*Underlined: site recognized by *Kpn*I

### For analysis of -69G → A mutation

5′-GAT GCA ACT GAT ACT TAC ACA ACC AG-3′ (Reverse: SEQ ID NO:
38)

### For analysis of L151P mutation

5′-ATG CCC CTG ACT GCT ACC TGT CTT TCC ATA AGC AGC-3′

(Forward: SEQ ID NO: 39)

*Underlined: mismatch base

### Detection of mutation in CETP gene

Mutations Int14 + 1G → A and D442G, which are types of mutation in CEPT gene identified at high incidence for Japanese hyperalphalipoproteinemic patients, were analyzed. In Int14 + 1G → A mutation, substitution of one base (GT) at the intron 14 splice donor site with AT is identified, and mRNA synthesized from the mutant allele does not allow normal splicing, to thereby fail to synthesize CETP (Brown M. L. *et al. Nature,* 342: 448-451, 1989). In D442G mutation, the base (A) at the 1403 position is substituted by G, changing the codon corresponding to amino acid residue 442 from GAC to GGC; i.e., amino acid residue at the 442 position is changed from aspartic acid (Asp: D) to glycine (Gly: G). It has been known that the serum CETP level is reduced by the mutation (Sakai N. *et al., Atherosclerosis,* 114: 139-146, 1995).

### Detection of Int14 + 1G → A by PCR-RFLP

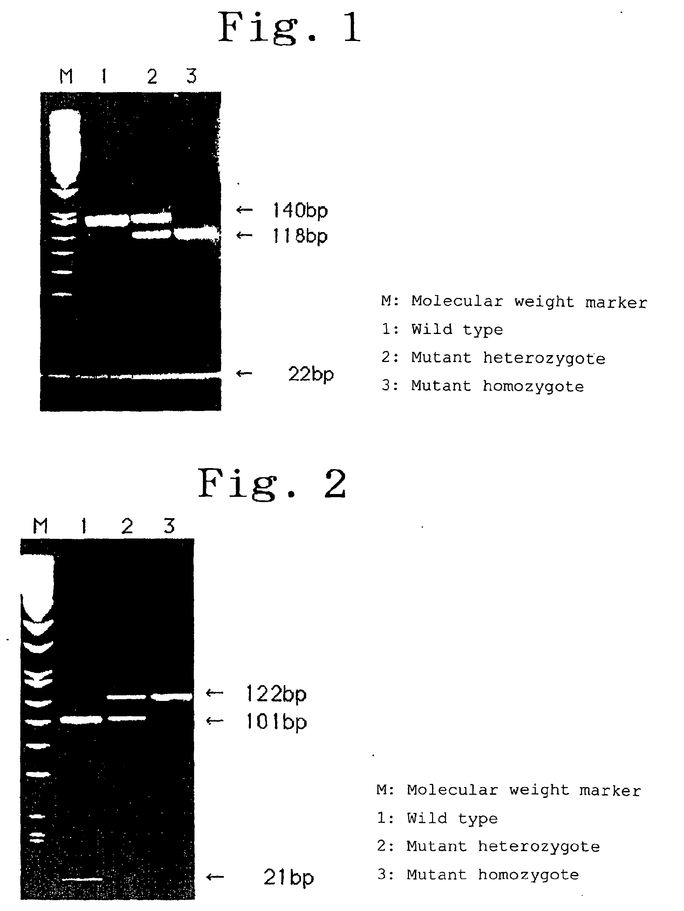

In Int14 + 1G → A mutation, the site recognized by the restriction enzyme does not change by substitution of a single base. However, when a mismatch base is introduced into an oligonucleotide primer on the reverse side, the mutation can be detected by PCR-RFLP. Genomic DNA (0.5 µg/µL) obtained from a subject through extraction was added to a PCR reaction mixture [10 mM Tris-HCl buffer (pH 8.3) containing 50 mM KCl and 1.5 mM MgCl₂] (50 µL) containing oligonucleotide primers (SEQ ID NOs: 3 and 4) (each 0.8 µM) and nucleotide triphosphates (dNTP) (each 200 µM), and *Taq* DNA polymerase (product of PERKIN ELMER) (0.5 units) was added to the resultant mixture for PCR. The PCR reaction was carried out under the following conditions: a cycle consisting of 94°C for 30 sec, 60°C for 30 sec, and 72°C for one minute; repetition of the cycle for 35 times; and 72°C for 10 minutes, to thereby yield a target PCR product of 140 bp. The PCR product yielded through PCR was identified by subjecting the PCR product (5 µL) to electrophoresis by use of 3% agarose in combination with staining by use of 0.5 µg/mL ethidium bromide. The PCR product (5 µL) was digested overnight at 37°C by use of a restriction enzyme *(Nde*I, product of Takara Shuzo Co., Ltd.) (5 units), followed by electrophoresis by use of 12.5% polyacrylamide gel, to thereby detect Int14 + 1G → A mutation. Specifically, when the gene was wild-type, no cleavage of the DNA fragment of 140 bp was observed, whereas when the gene had Intl4 + 1G → A mutation, the DNA fragment was cleaved into a fragment of 118 bp and a fragment of 22 bp. The mutation can be identified on the basis of the results (Fig. 1).

### Detection of D442G mutation by PCR-RFLP

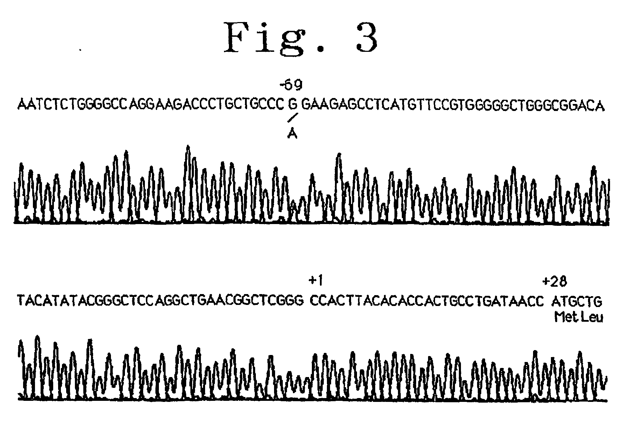

In D442G mutation, the site recognized by the restriction enzyme does not change. However, when a mismatch base is introduced into an oligonucleotide primer on the forward side, the mutation can be detected by PCR-RFLP. Genomic DNA (0.5 µg/µL) obtained from a subject through extraction was subjected to PCR in the aforementioned manner, except that oligonucleotide primers (SEQ ID NOs: 5 and 6) were used, to thereby yield a target PCR product of 122 bp. The PCR product (5 µL) was digested overnight at 37°C by use of a restriction enzyme *(Sal*I, product of Toyobo) (4 units), followed by electrophoresis by use of 12.5% polyacrylamide gel, to thereby detect D442G mutation. Specifically, when the gene was wild-type, the DNA fragment of 122 bp was cleaved into a fragment of 101 bp and a fragment of 21 bp, whereas when the gene had D442G mutation, no cleavage of the DNA fragment was observed. The mutation can be identified on the basis of the results (Fig. 2).

### Analysis of Int14 + 1G → A mutation and D442G mutation occurring in 13 subject cases

Through PCR-RFLP, occurrence of Int14 + 1G → A mutation and that of D442G mutation were analyzed. Eight (cases 1-8) out of nine cases, having exhibited a serum CETP activity considerably reduced to 0-5% (compared to the average of healthy controls), were identified as homozygotes of Int14 + 1G → A mutation, and case 9 was identified as a heterozygote of Int14 + 1G → A mutation. Case 10, having exhibited a CETP activity of 25%, was identified as a compound heterozygote in terms of Int14 + 1G → A mutation and D442G mutation; i.e., a complex heterozygote. The three remaining cases (cases 11, 12, and 13), having exhibited a CETP activity of 30-40%, were identified as heterozygotes of D442G mutation.

The average serum CETP activity of eight heterozygotes (4 male and 4 female) with Intl4 + 1G → A mutation and 21 heterozygotes (13 male and 8 female) with D442G mutation were found to be 59 ± 19% and 82 ± 23% (average ± standard deviation), respectively. Accordingly, 4 cases (cases 9, 11, 12, and 13) are considered to bear a mutation other than the aforementioned two types of the CETP gene mutation. Thus, the nucleotide sequences of all exons of the CETP gene were analyzed for these four cases.

### Analysis of all exons in CETP gene

### PCR amplification

Each exon of the CETP gene was amplified through PCR. Specifically, genomic DNA (0.5 µg/µL) obtained from subjects through extraction was added to a PCR reaction mixture (50 µL) containing synthetic oligonucleotide primers (each 0.8 µM) and dNTPs (each 200 µM), and *Taq* DNA polymerase (0.5 units) was added to the resultant mixture for PCR. The PCR reaction was carried out under the following conditions: a cycle consisting of 94°C for 30 sec, 60°C for 30 sec, and 72°C for one minute and 30 sec; repetition of the cycle for 35 times; and 72°C for 10 minutes, to thereby yield a target PCR product. The PCR product yielded through PCR was identified by subjecting the PCR product (5 µL) to electrophoresis by use of 3% agarose gel in combination with staining by use of 0.5 µg/mL ethidium bromide.

### Determination of nucleotide sequence

Each of the thus-amplified PCR products was analyzed in terms of nucleotide sequence, through direct sequencing by use of an auto-sequencer. Specifically, each PCR product of the corresponding exon was fluorescence-labeled by use of a "Big Dye Terminator cycle sequencing Fs Ready Reaction Kit" (product of PERKIN ELMER) and subsequently, the nucleotide sequence was determined by means of an "ABI PRISM 377 DNA Sequencer" (product of APPLIED BIOSYSTEMS).

The PCR product corresponding to exon 7 of case 9 was sub-cloned by use of a TA Cloning Kit (product of INVITROGEN), and the nucleotide sequence of the resultant clone was determined in a manner similar to that described above.

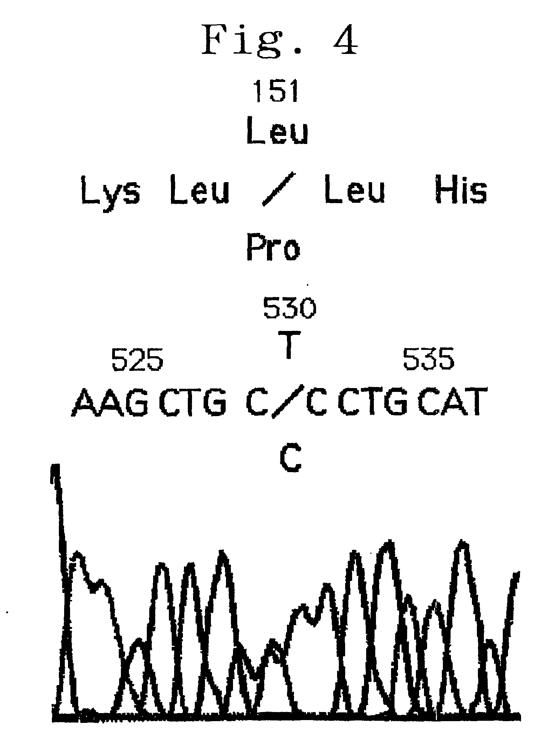

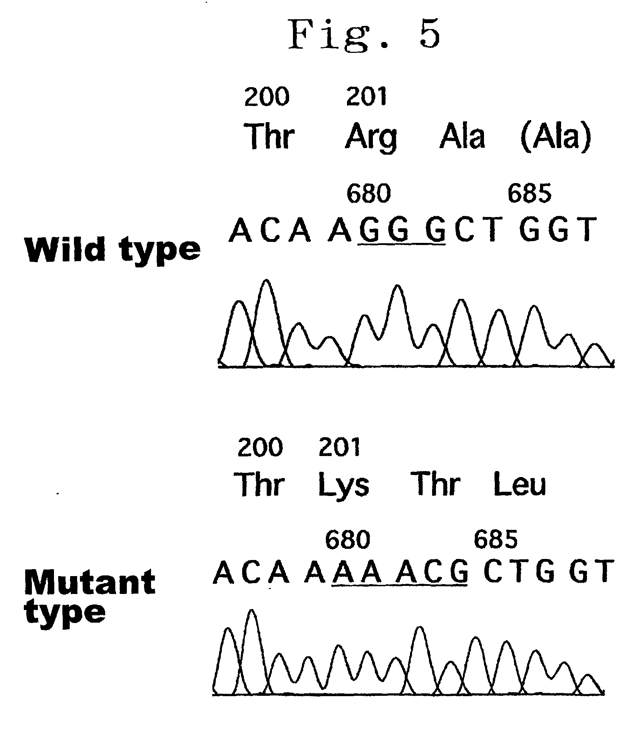

The results of the nucleotide sequence analysis indicate that a specific base substitution was identified for each of four cases in addition to a heterozygote of Int14 + 1G → A mutation or D442G mutation and I405V polymorphism (Agellon, L. B. *et al., Biochemistry,* 29: 1372-1376, 1990). Specifically, in case 11, nucleotide A―other than nucleotide G of the wild-type gene―was detected at the - 69-position of the transcription regulatory region [69 base pair (bp) upstream of the start codon to be transcribed to mRNA] (hereinafter also referred to as -69G → A mutation) (Fig. 3). In case 12, nucleotide C―other than nucleotide T of the wild-type gene―was detected at the 530-position of the CETP gene. By substitution of this T by C, the codon for amino acid residue 151 was identified as having been altered from CTC to CCC, resulting in substitution of leucine (Leu: L)―amino acid residue 151―with proline (Pro: P) (hereinafter also referred to as L151P mutation) (Fig. 4). In case 9, in addition to clones having a wild-type nucleotide sequence, there was detected a clone in which wild-type GGG corresponding to the 680-682 positions are deleted and AAACG are newly inserted. By substitution of this GGG with AAACG, the codon for the amino acid residues 201 and 202 was identified to be altered from AGG/GCT to AAA/ACG, resulting in substitution of arginine (Arg: R)/alanine (Ala: A)―amino acid residues 201 and 202―with lysine (Lys: K)/threonine (Thr: T). In addition, alteration of amino acid residue 218 to a stop codon attributable to reading frame shift in connection with subsequent amino acid residues was identified (hereinafter also referred to as 680 del GGG/ins AAACG mutation) (Fig. 5).

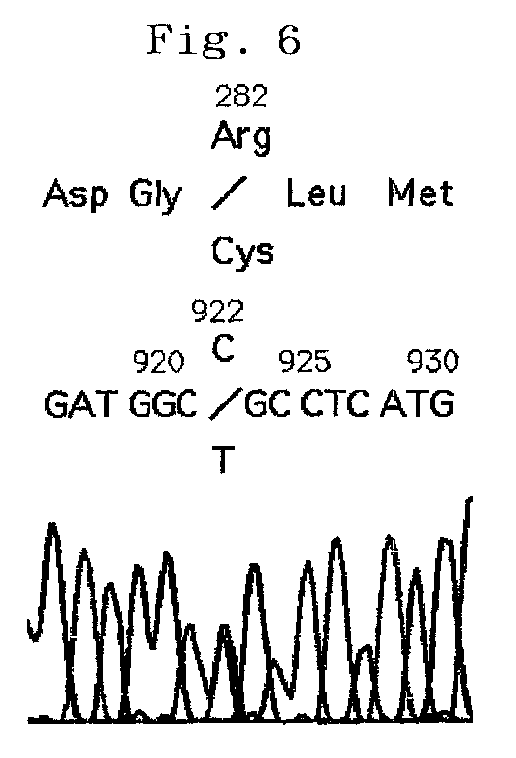

In case 13, nucleotide T―other than nucleotide C of the wild-type gene―was detected at the 922-position of the CETP gene. By substitution of this C with T, the codon for amino acid residue 282 was identified as having been altered from CGC to TGC, resulting in substitution of arginine (Arg: R)―amino acid residue 282―with cystein (Cys: C) (hereinafter also referred to as R282C mutation) (Fig. 6).

Among these four types of nucleotide substitution, the mutation -69G → A was found to be a type of nucleotide substitution detected in the sequence of a PEA3 motif (consensus sequence; CAGGAAGT)―a nucleotide sequence to be linked to an ETS gene family serving as a transcription factor (Oliveira, H. C. F et *al., J. Biol. Chem.,* 271: 31831-31838, 1996). In the consensus sequence of the PEA3 motif, the sequence -GGAA- is a highly conserved one, and substitution of one base in CAGGAAGT to form CAAGAAGT has been known to lead to failure of binding with ETS1 (Fisher R. J. *et al., J. Biol*. *Chem*., 267: 17957-17965, 1992). Thus, one conceivable reason for suppression or reduction of expression of CETP is that -69G → A mutation detected in the transcription regulatory region in the CETP gene inhibits binding to a nuclear transcription factor, thereby suppressing synthesis of mRNA.

In 680 del GGG/ins AAACG mutation, because shifting of amino acid reading frame, the amino acid sequence from 201 to 217 is altered to an amino acid sequence entirely different from that of normal CETP, and amino acid residue 218 is altered to a stop codon. Since the serum CETP activity of a homozygote of G181X mutation (i.e., nonsense mutation in which amino acid residue 181 is altered to a stop codon) is completely lost (Arai T., *J. Lipid Res.,* 37: 2145-2154, 1996), occurrence of 680 del GGG/ins AAACG mutation is also conceived to defect synthesis of active CETP.

L151P mutation and R282C mutation are so-called missense mutation involving substitution of amino acid residues. Since the three-dimensional structure of synthesized CETP is changed by these two types of mutation, it is conceived that mutant CETP is not secreted to the circulation or that mutant CETP loses its activity (CE transfer activity) even though it is secreted to the circulation.

The effect of -69G → A mutation on transcription activity of a CETP gene has been investigated.

### Measurement of transcription activity of CETP gene

A transcription regulatory region of a CETP gene was ligated to an upstream portion of a luciferase gene serving as a reporter gene. The reporter gene was inserted in Hep G2 cells originating from hepatic cells, and the amount of expressed luciferase was evaluated as transcription activity of the CETP gene. Transcription activity was measured by either of two reporter genes; i.e., a reporter gene constructed with nucelotides at the -1 position to the -570 position of the transcription regulatory region of the CETP gene and a reporter gene constructed with nucelotides at the -1 position to the -164 position of the transcription regulatory region of the CETP gene.

### Production of plasmid DNA for measuring transcription activity

Genomic DNA (0.5 µg/µL) obtained from case 11 in which -69G → A base substitution had been detected and PCR was carried out in the aforementioned manner, by use of an oligonucleotide primer (SEQ ID NO: 35) and a similar primer (SEQ ID NO: 36) in which a site recognized by restriction enzyme *Xho*I had been introduced, to thereby yield a target PCR product of 845 bp. The PCR product was digested by use of restriction enzymes *Kpn*I and *Xho*I, and the resultant fragment of 564 bp was inserted to the *Kpn*I/*Xho*I site of a PicaGene basic vector 2 (product of Wako Pure Chemicals Industries, Ltd.) by use of a DNA Ligation Kit (product of Takara Shuzo Co., Ltd.). By use of the integrated vector, *E. coli* JM109 competent cells (product of Toyobo) were transformed. Among the transformed cells, ampicillin-resistant cells were selected, and the plasmid DNA was isolated by use of a QIAGEN Plasmid Mini Kit (product of QIAGEN). The nucleotide sequence of the transcription regulatory region which had been inserted into the vector was identified by use of the purified plasmid DNA. Thus, a pCETP-570 vector (wild type) having a wild-type sequence and a pCETP-570 (-69A) vector (mutant type) having -69G → A mutation were produced. In a similar manner, PCR was performed by use of oligonucleotide primer (SEQ ID NO: 37) in which a site recognized by restriction enzyme *Kpn*I had been introduced and the primer (SEQ ID NO: 36), to thereby yield a PCR product of 182 bp. The PCR product was digested by use of restriction enzymes *Kpn*I and *Xho*I, and the resultant fragment of 158 bp was inserted to a PicaGene basic vector 2 in a manner similar to that described above, to thereby produce a pCETP-164 vector (wild type) and a pCETP-164 (-69A) vector (mutant type).

### Measurement of luciferase activity

Hep G2 cells were cultured in Dulbecco's modified Eagle's minimum essential medium (DMEM) containing 10% fetal bovine serum and penicillin/streptomycin (10 units/mL), at 37°C under 5% CO₂ incubator conditions. One day before transfection, the cells were seeded in a 6-well microplate (product of Iwaki Glass Company, Ltd.) at 1 × 10⁵/well. After completion of culture for 24 hours, Hep G2 cells were transfected with each of the reporter vectors (1 µg each) by use of a FuGENE™ 6 transfection Reagent (product of BOEHRRINGER MANNHEIM). The cells were also transfected with pCMV-β-gal plasmid simultaneously. After completion of another culture for 24 hours, a cell lysate was prepared by use of a PicaGene cultured cell cytolytic agent (LCB, product of Wako Pure Chemicals Industries, Ltd.) (250 µL). Luciferase activity was measured by use of a PicaGene luciferase assay system (product of Wako Pure Chemicals Industries, Ltd.) and a luminometer (Lumat LB9501, product of BERTHOLD). In addition, β-galactosidase activity was measured by use of AURORA™ Gal-XE (product of ICN). The transcription activity of each vector was calculated as luciferase activity per unit of β-galactosidase activity (RLU/unit).

### Effect of -69G → A base substitution on transcription activity

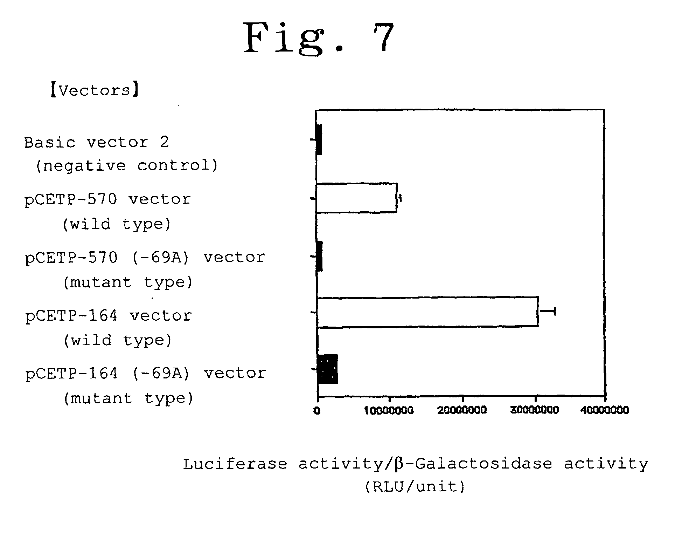

The transcription activity of the pCETP-570 (-69A) vector (mutant type) was found to be 0.3% based on that of the pCETP-570 vector (wild type), indicating substantially complete suppression of transcription activity. In the case of the pCETP-164 (-69A) vector (mutant type), the activity was considerably reduced to 6.5% relative to that of the wild type vector (Fig. 7).

The results indicated that the detected and identified -69G → A mutation considerably lowers the transcription activity of the CETP gene, to thereby suppress synthesis of mRNA, leading to reduction in serum CETP level (activity).

### Analysis of frequency of novel CETP gene mutation

The frequency of any of the aforementioned mutations detected and identified in the CETP gene; i.e., -69G → A mutation; L151P mutation; 680 del GGG/ins AAACG mutation; or R282C mutation, was investigated for hyperalphacholesterolemic patients who had a serum HDL-C level of 100 mg/dL or more and agreed to receive the gene analysis of the present invention. Since the site recognized by a restriction enzyme varies by base substitution of -69G → A mutation; 680 del GGG/ins AAACG mutation; or R282C mutation, these types of mutation were identified by PCR-RFLP. When L151P mutation causes substitution of one base pair, the site recognized by the restriction enzyme does not change. However, when a mismatch base is introduced into an oligonucleotide primer on the forward side, the mutation can be detected by PCR-RFLP.

### Detection of -69G → A mutation through PCR-RFLP

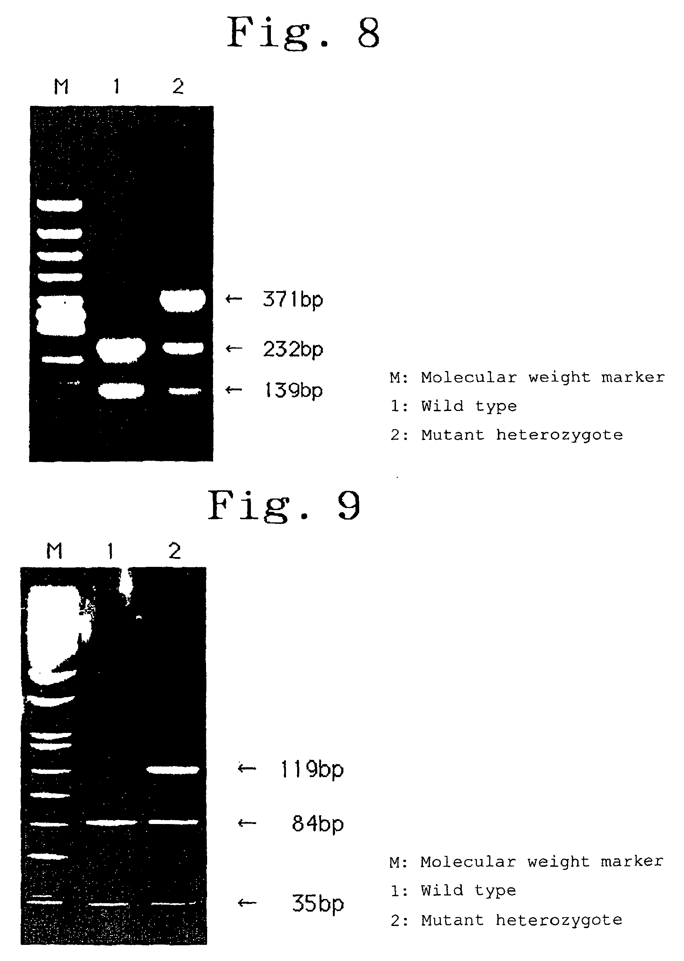

Genomic DNA (0.5 µg/µL) obtained from a subject through extraction was subjected to PCR in the aforementioned manner, by use of oligonucleotide primers (SEQ ID NOs: 7 and 38), to thereby yield a target PCR product of 371 bp. The PCR product (5 µL) was digested overnight at 37°C by use of a restriction enzyme (*Hap*II, product of Takara Shuzo Co., Ltd.) (4 units), followed by electrophoresis by use of 3% agarose, to thereby detect -69G → A mutation. Specifically, when the gene was wild-type, the DNA fragment of 371 bp was cleaved into a fragment of 232 bp and a fragment of 139 bp, whereas when the gene had -69G → A mutation, no cleavage of the DNA fragment was observed. The mutation can be identified on the basis of the results (Fig. 8).

### Detection of L151P mutation through PCR-RFLP

Genomic DNA (0.5 µg/µL) obtained from a subject through extraction was subjected to PCR in the aforementioned manner, by use of oligonucleotide primers (SEQ ID NOs: 39 and 14), to thereby yield a target PCR product of 119 bp. The PCR product (5 µL) was digested overnight at 37°C by use of a restriction enzyme (*Alu*I, product of Toyobo) (4 units), followed by electrophoresis by use of 12.5% polyacrylamide gel, to thereby detect L151P mutation. Specifically, when the gene was wild-type, the DNA fragment of 119 bp was cleaved into a fragment of 84 bp and a fragment of 35 bp, whereas when the gene had L151P mutation, no cleavage of the DNA fragment was observed. The mutation can be identified on the basis of the results (Fig. 9).

### Detection of 680 del GGG/ins AAACG mutation through PCR-RFLP

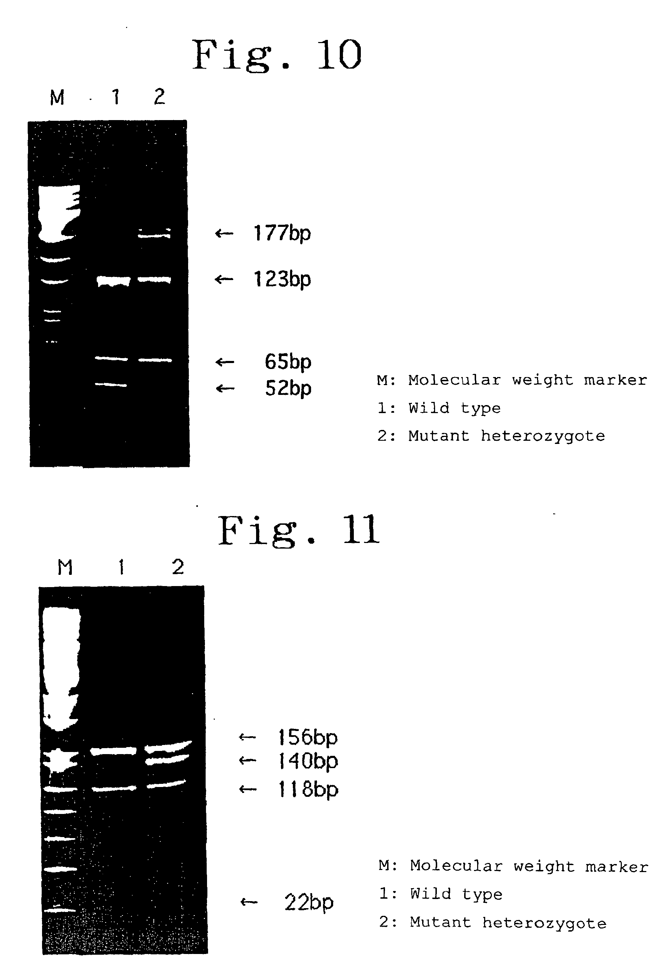

Genomic DNA (0.5 µg/µL) obtained from a subject through extraction was subjected to PCR in the aforementioned manner, by use of oligonucleotide primers (SEQ ID NOs: 17 and 18), to thereby yield a target PCR product of 240 bp (mutant: 242 bp). The PCR product (5 µL) was digested overnight at 37°C by use of a restriction enzyme (*Xcm*I, product of Daiichi Kagaku) (4 units), followed by electrophoresis by use of 12.5% polyacrylamide gel, to thereby detect 680 del GGG/ins AAACG mutation. Specifically, when the gene was wild-type, the DNA fragment of 240 bp was cleaved into three fragments; i.e., a fragment of 123 bp, a fragment of 65 bp, and a fragment of 52 bp, whereas when the gene had 680 del GGG/ins AAACG mutation, the DNA fragment of 242 bp was cleaved into a fragment of 177 bp and a fragment of 65 bp. The mutation can be identified on the basis of the results (Fig. 10).

### Detection of R282C mutation by PCR-RFLP

Genomic DNA (0.5 µg/µL) obtained from a subject through extraction was subjected to PCR in the aforementioned manner, by use of oligonucleotide primers (SEQ ID NOs: 17 and 18), to thereby yield a target PCR product of 296 bp. The PCR product (5 µL) was digested overnight at 37°C by use of a restriction enzyme (*Hae*III, product of Takara Shuzo Co., Ltd.) (3 units), followed by electrophoresis by use of 12.5% polyacrylamide gel, to thereby detects R282C mutation. Specifically, when the gene was wild-type, the DNA fragment of 296 bp was cleaved into three fragments; i.e., a fragment of 156 bp, a fragment of 118 bp, and a fragment of 22 bp, whereas when the gene had R282C mutation, the DNA fragment of 296 bp was cleaved into a fragment of 156 bp and a fragment of 140 bp. The mutation can be identified on the basis of the results (Fig. 11).

### Frequency of mutations occurring in hyperalphacholesterolemic patients

The frequency of any of the mutations; i.e., -69G → A mutation; L151P mutation; 680 del GGG/ins AAACG mutation; or R282C mutation was analyzed for 117 hyperalphacholesterolemic patients who had a serum HDL-C level of 100 mg/dL or more. The results indicate that a heterozygote of -69G → A mutation was identified in four cases (including case 11), and the frequency of the mutation was found to be 3.4%. L151P mutation, 680 del GGG/ins AAACG mutation, and R282C mutation were identified only in cases 12, 9, and 13, respectively in which each mutation had originally been identified, and the frequency of each mutation was found to be 0.9%.

As mentioned hereinabove, four types of new mutation; i.e., -69G → A mutation; L151P mutation, 680 del GGG/ins AAACG mutation, and R282C mutation, were identified among 13 hyperalphacholesterolemic patients. Particularly, -69G → A mutation occurs at a frequency lower than that of conventionally identified Int14 + 1G → A mutation, but at a frequency higher than those of other mutations which have already been reported. Thus, the frequency of the -69G → A mutation is conceived to be the second to that of Int14 + 1G → A mutation.

In addition, as described above, the -69G → A mutation occurs in the transcription regulatory region of the CETP gene, and transcription activity is considered to be significantly reduced through substitution of G by A. In fact, subjects having the gene mutation have been identified. This transcription regulatory region clearly plays an important role for expression of CETP. Thus, it has been proven that the serum CETP level can be controlled by regulation of this region. In other words, substances which act on this region, or substances which provide such former substances are expected to elevate serum CETP level. In addition, the serum CETP level of a number of heterozygote patients who are completely CETP deficient is expected to elevate through the regulation on this region of a normal allele. Furthermore, the serum CETP level of subjects having the -69G → A mutation can be elevated by use of a certain substance which acts on the mutant sequence.

For subjects with over-expression of CETP, their serum CETP may be controlled through reverse action to the aforementioned action. In addition, those who are completely CETP deficient as identified through the detection method of the present invention can be confirmed as so-called gene therapy targets.

Although the L151P mutation, 680 del GGG/ins AAACG mutation, and R282C mutation occur at a low frequency, combination of these types mutation with the -69G → A mutation, Int14 + 1G → A mutation, or other known minor mutations contributes to enhancement of the effect of identifying a risk factor for the onset of arteriosclerosis caused by abnormality of CETP.

### Industrial Applicability

According to the present invention, a novel, high-frequent mutation which causes CETP deficiency can be identified, and means for preventing and retarding development of arteriosclerosis on the basis of the finding on the mutation can be provided.

## Claims

1. A method of detecting arteriosclerosis comprising detecting a risk factor for the onset of arteriosclerosis in a subject on the basis of correlation between mutation in a CETP gene and risk of arteriosclerosis.

2. A method of detecting arteriosclerosis as described in claim 1, wherein the mutation is identified at one or more sites selected from among (1) guanine at the -69-position of the CETP gene; (2) the site encoding leucine at the 151-position of CETP encoded by the CETP gene; (3) the site encoding arginine at the 201-position and alanine at the 202-position of the same; and (4) the site encoding arginine at the 282-position of the same.
